# EUROPEAN PATENT APPLICATION

(11) **EP 2 586 381 A1**
(43) Date of publication of application: **01.05.2013**
(21) Application number: 12186176.9
(22) Date of filing: 26.09.2012
(51) Int. Cl.: A61B 17/072

(54) **Surgical apparatus for endoluminal surgery**

(30) Priority: 25.10.2011 US 201113280871
(71) Applicant: Covidien LP, Mansfield, MA 02048 (US)
(72) Inventor: Aranyi, Ernest, Easton, CT Connecticut 06612 (US); Racenet, David, Middletown, CT Connecticut 06457 (US); Bronson, Dwight, Cheshire, CT Connecticut 06410 (US)
(74) Representative: Maschio, Antonio

(57) **Abstract**

The present disclosure is directed to an endoscopic surgical instrument and methods for performing a diverticulum treatment. The surgical instrument includes a handle assembly, an elongated member and a jaw assembly. The elongated member is operably coupled to the distal end of the handle assembly, while the jaw assembly is operably coupled to a distal end of the elongated member. The jaw assembly includes a knife slot that is defined therewithin and is adapted to receive a knife blade to thereby cut tissue that is disposed between the jaw assembly. The jaw assembly is configured to approximate an esophageal tract and a diverticulum.

## Description

### BACKGROUND

### Technical Field

The present disclosure relates to an apparatus and method of performing a surgical procedure. More particularly, the present disclosure relates to an apparatus and method for performing an endolumenal surgical procedure.

### Background of Related Art

In an endoluminal surgery, a surgical instrument is inserted into a patient's body through a naturally occurring orifice. These procedures include esophageal procedures, such as a procedure for correcting Zenker's diverticulum. In anatomy, a diverticulum is a blind sac that branches off from a cavity of the body. A Zenker's diverticulum, which is also referred to as a pharyngoesophageal diverticulum, is a pouch that develops in the mucosa tissue of the pharynx above the circopharyngeal muscle. It is the most common type of esophageal diverticulum and usually occurs to elderly patients. When a patient having a Zenker's diverticulum swallows, some food ends up in the pouch until the pouch fills. Once the pouch is filled, the pouch presses on the upper esophagus, making it difficult for solid foods to pass through the esophagus. The filled pouch also spills food into the throat, causing coughing and spitting up of food that has been previously swallowed, sometimes many hours before. The pouch also causes problems for patients taking medication (e.g., pills) since the medication may become trapped within the pouch, which prevents the medication from being absorbed through the stomach.

Currently, there are two approaches to treating Zenker's diverticulum in patients, conventional open surgery and endoscopic surgery. The open surgical approach involves making an incision through the neck, locating the pouch behind the lower throat, cutting the cricopharyngeus muscle that forms the top of the pouch and cutting out the pouch or tacking it upside down so that it cannot fill. The open surgical approach, however, requires a day or two in the hospital.

The endoluminal surgical approach is performed via the mouth using rigid metal tubes. A laser or stapling device may be used to divide the cricopharyngeus muscle and the wall between the pouch and the esophagus. This approach eliminates the pouch by making it part of the upper esophagus. Compared to open surgery, the endolumenal approach is faster, equally effective at relieving symptoms, and has a lower complication rate. However, there exists a need for devices that perform a quicker and more efficient minimally invasive surgical procedure than the devices currently used in the art. It is also desirable that the instrumentation utilized in the surgery does not harm the esophageal tissue as it is inserted through the mouth and down the esophagus.

### SUMMARY

The present disclosure relates to an endoscopic surgical instrument, which includes various types of endoscopic instrumentation.

A surgical instrument for performing an endolumenal surgical procedure, comprising: a handle assembly, an elongated member operably coupled to the distal end of the handle assembly and a jaw assembly having a pair of jaw members, one or both of the jaw members defining a knife slot adapted to receive a knife blade, one of the pair of jaw members including an anvil and the other of the pair of jaw members including a staple cartridge, the pair of jaw members being movable relative to one another so that the anvil and staple cartridge can be moved to an approximated position, the anvil and staple cartridge each having distal ends, the distal end of the staple cartridge extending distally beyond the distal end of the anvil.

In certain embodiments, the elongated member and jaw assembly are configured to be inserted into the esophagus of a patient. The jaw assembly may be configured to engage a portion of the esophagus to treat a diverticulum in the esophagus. The elongated member may be flexible, rigid, have flexible and rigid portions, or include an articulating portion. The instrument can further comprise an endoscopic camera that is operably coupled to a distal end of at least one of the jaw members. The endoscopic camera may be positioned on the at least one of the jaw members to provide an endoscopic view of the esophageal tract.

In certain embodiments, the camera is positioned adjacent the anvil. The distal end of the anvil forms a blunt tip, in certain desirable embodiments.

The knife slot desirably terminates at position substantially aligned with a distal-most fastener forming recess of the anvil.

### BRIEF DESCRIPTION OF THE DRAWINGS:

The above and other aspects, features, and advantages of the present disclosure will become more apparent in light of the following detailed description when taken in conjunction with the accompanying drawings in which:

Fig. 1 is a rear, perspective view of a surgical instrument according to the present disclosure;

Fig. 2A is a rear, perspective view of an elongated member for a surgical instrument according to another embodiment, the elongated member having a rigid portion and a flexible portion;

Fig. 2B is a schematic illustration of a handle assembly for a surgical instrument in accordance with the embodiment of Fig. 2A;

Fig. 3 is a perspective view of a jaw assembly in accordance with the embodiment of Figs. 2A and 2B;

Figs. 4 and 5 are bottom, perspective views of the jaw assembly in accordance with the embodiment of Figs. 2A, 2B and 3;

Fig. 6 is an exploded perspective view of a jaw assembly according to another embodiment of the present disclosure;

Fig. 6b is an exploded perspective view of a jaw assembly according to the embodiment of Fig. 6;

Fig. 7 is a cross-sectional view of the jaw assembly according to the embodiment of Figs. 6 and 6a;

Fig. 8 is a partial cross-sectional view of the jaw assembly according to the embodiment of Figs. 6 through 7;

Fig. 9 is a perspective view of the jaw assembly, with parts removed, according to the embodiment of Figs. 6 through 8;

Fig. 10 is a perspective view of the jaw assembly, with parts removed, according to the embodiment of Figs. 6 through 9;

Fig. 11 is an exploded perspective view of drive assembly according to the embodiment of Figs. 6 through 10

Fig. 12 is a perspective view of an esophagus having a Zenker's diverticulum;

Fig. 13 is a side, cross-sectional view of the esophagus as taken through 13-13 of Fig. 12;

Figs. 14 and 15 are top, cross-sectional views of the indicated areas of Fig. 13;

Figs. 16-18 illustrate a method of treating an esophagus having a Zenker's diverticulum with another exemplary endoscopic instrument in accordance with the present disclosure;

Fig. 19 is a perspective view of an esophagus that has undergone a Zenker's diverticulum treatment in accordance with the present disclosure;

Fig. 20 is a top, cross-sectional view of the treated esophagus of Fig. 19;

Fig. 21 is a side, cross-sectional view of the treated esophagus of Fig. 19 as taken along the line 21-21 in Fig. 19;

Fig. 22 is a side, cross-sectional view of handle assembly according to a further embodiment of the present disclosure;

Fig. 23 is a top, cross-sectional view in accordance with the embodiment of Fig. 30;

Fig. 24 is a front, perspective view of the handle assembly with the elongated member separated therefrom, in accordance with the embodiment of Figs. 30 through 31;

Figs. 25 and 26 are side perspective views of a jaw assembly according to the embodiment of Figs. 22 through 24;

Fig. 27 is an exploded, perspective view of the jaw assembly in accordance with the embodiment of Figs. 22 through 26;

Fig. 28 is a side, cross-sectional view of the jaw assembly in accordance with the embodiment of Figs. 22 through 27; and

Fig. 29 is a top, cross-sectional view of the jaw assembly in accordance with the embodiment of Figs. 22 through 28.

Other features of the present disclosure will become apparent from the following detailed description, taken in conjunction with the accompanying drawings, which illustrate, by way of example, the principles of the present disclosure.

### DETAILED DESCRIPTION

Embodiments of the presently disclosed surgical devices are described in detail with reference to the drawings, in which like reference numerals designate identical or corresponding elements in each of the several views. As used herein, the term "distal" refers to that portion of the devices, or component thereof, farther from the user while the term "proximal" refers to that portion of the devices or component thereof, closer to the user.

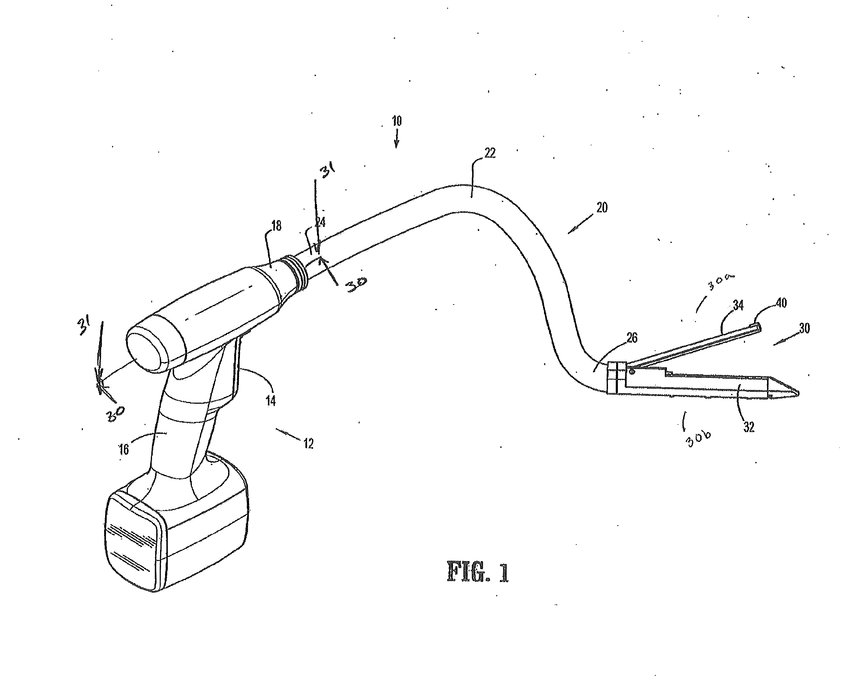

Referring now to Fig. 1, an endoscopic instrument having a jaw assembly and an elongated member is shown. The jaw assembly, in certain embodiments disclosed herein, is attached to an elongated shaft member that is integral with, or attached to the handle assembly of a surgical instrument. The elongated shaft member may be rigid, or flexible, or have rigid and flexible portions, or include one or more pivotable pivot members.

The elongated member may be articulated by the user of the instrument. In embodiments, the handle assembly and elongated member may include any suitable articulating mechanisms known in the art to manipulate (e.g., rotate and articulate) elongated member, which include, but not limited to gears, wires, cables, linkages, tubular shafts, drive rods, and combinations thereof.

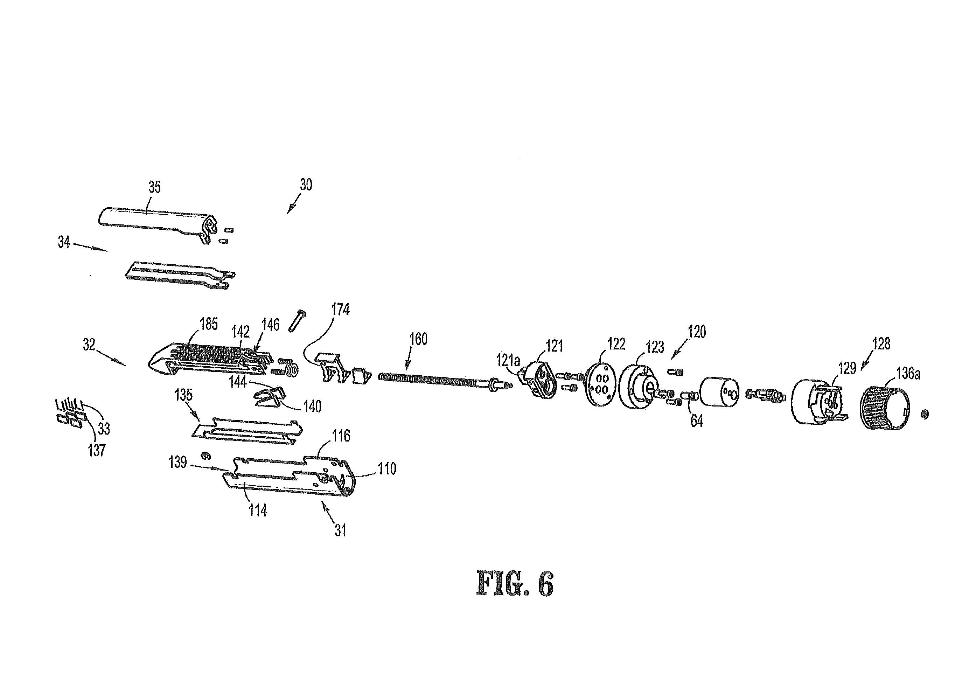

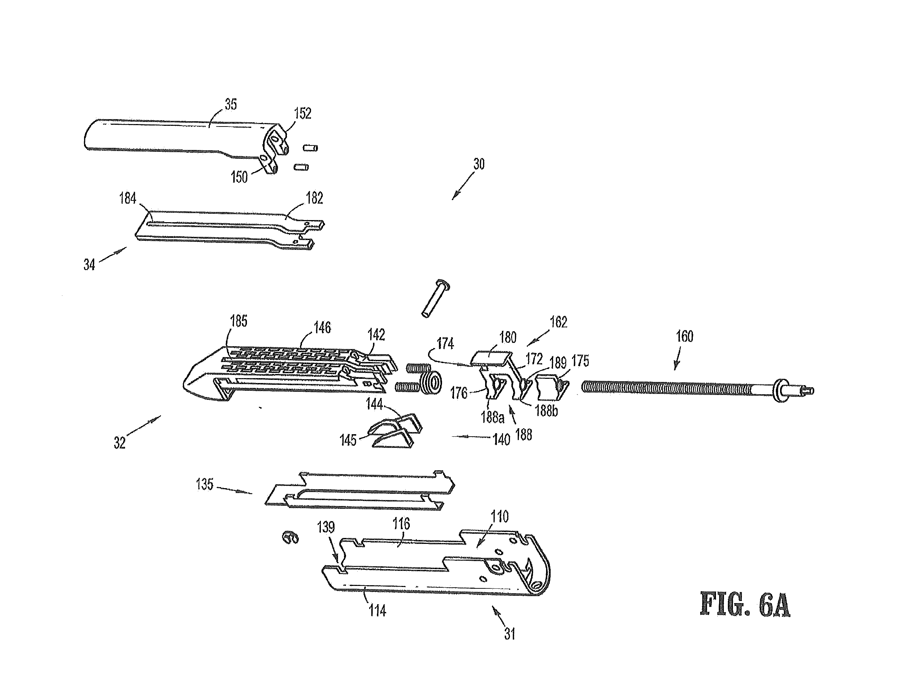

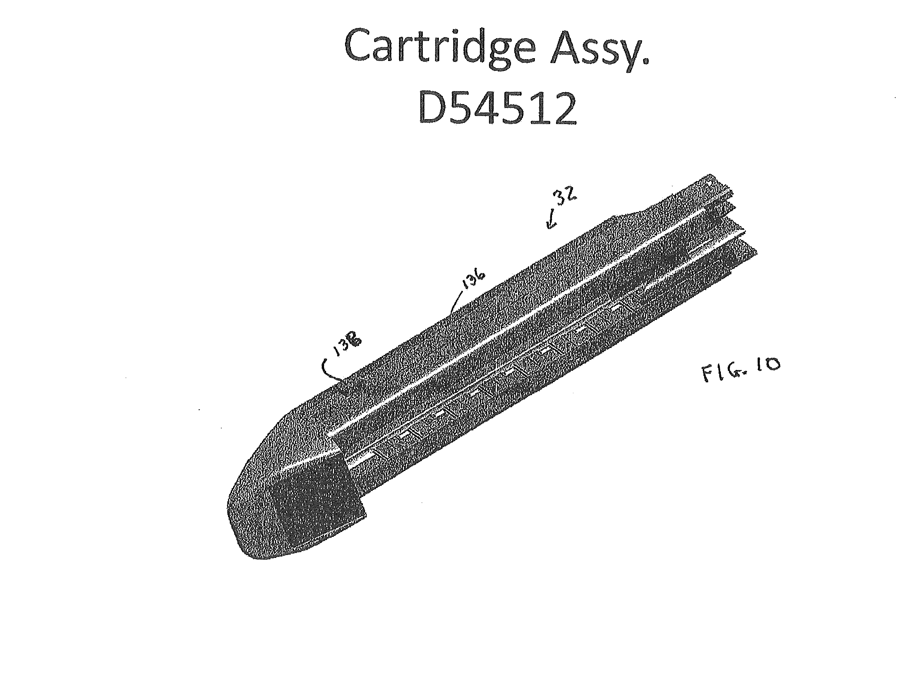

Referring now to Fig. 1, an endoscopic instrument 10 is shown. Endoscopic instrument 10 includes an elongated member 20 that is operably coupled to handle assembly 12, as shown in Fig. 1. Handle assembly 12 includes a housing 16 and an actuator 14 for actuating jaw assembly 30 between an open and a closed position. Actuator 14 also provides activation of stapling and cutting mechanisms to thereby fasten and cut tissue disposed between the jaw assembly 30, which may be as described in more detail below with respect to Figs. 6-11 or 26-29.

As shown in Fig. 1, elongated member or shaft 20 includes a flexible portion 22 having proximal and distal ends 24 and 26, respectively. Proximal end 24 of flexible portion 22 is operably coupled to a joining portion 18 of handle assembly 12, while distal end 26 of flexible portion 22 is operably coupled to jaw assembly 30. In embodiments, elongated member or shaft 20 may be articulated by manipulation of another actuator of the handle assembly 12 or by any other switching mechanism (not shown) that may be provided on handle assembly 12. Alternatively or additionally, the elongated member 20 may be manually articulated by a user. In embodiments, handle assembly 12 and elongated member 20 may include any suitable articulating mechanisms known in the art to manipulate (e.g., rotate and articulate) elongated member 20, which include, but not limited to gears, wires, cables, linkages, tubular shafts, drive rods, and combinations thereof.

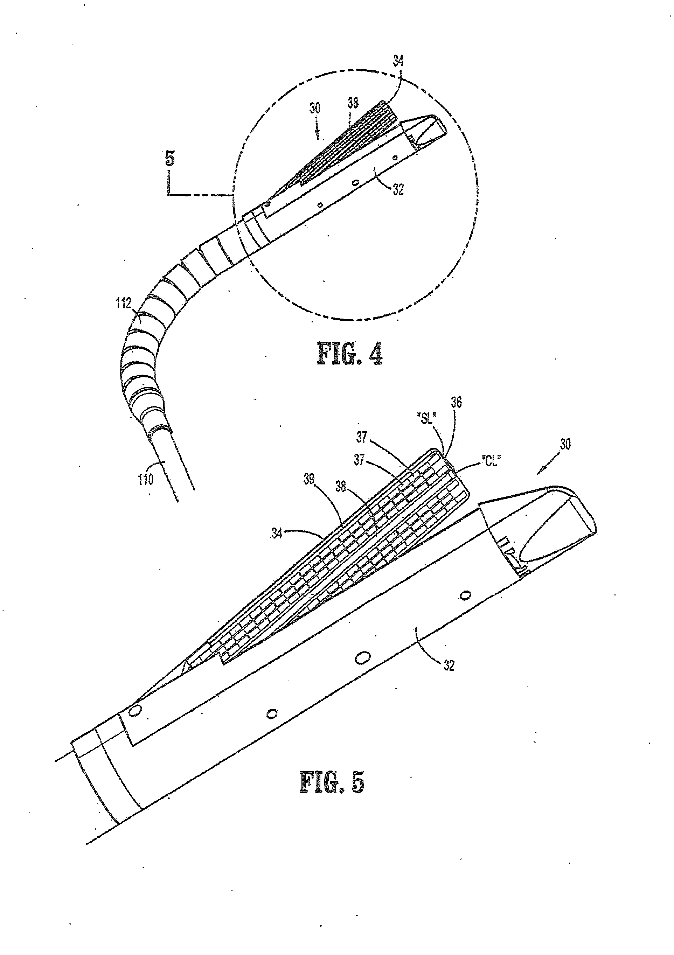

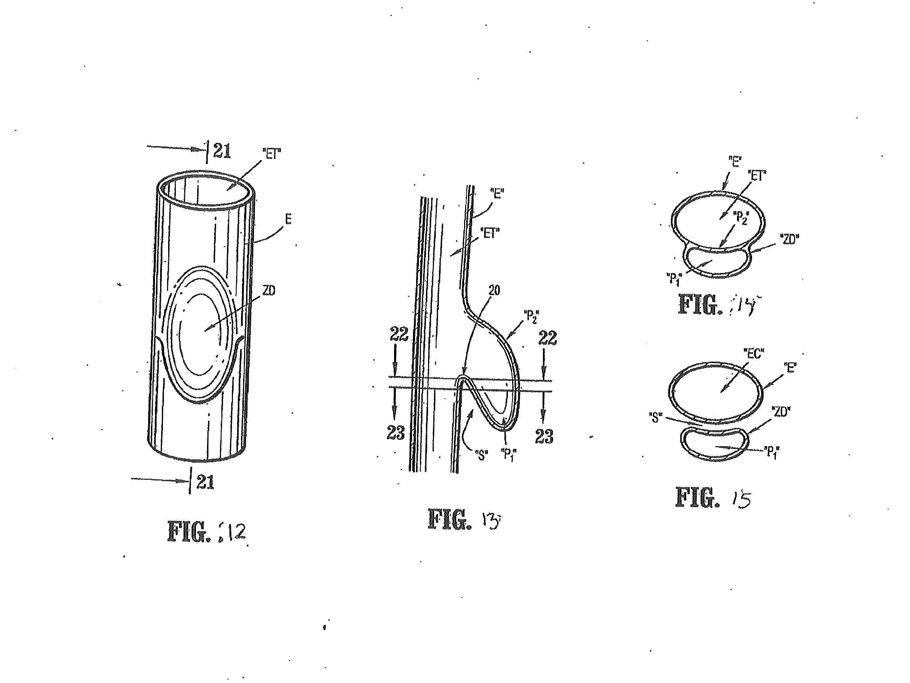

Jaw assembly 30 includes a pair of jaw members, 30a and 30b, which include a staple cartridge assembly 32 and an anvil 34. Cartridge assembly 32 houses one or more fasteners or staples 33 (Fig. 14 and 15) that are disposed therewithin and is configured to deploy the one or more fasteners or staples 33 upon actuation of actuator 14 by a user. Cartridge assembly 32 is mounted on a distal end 26 of elongated member 20. The pair of jaw members are movable relative to one another so that the anvil and/or cartridge can be moved into an approximated position. Anvil 34 is movably (e.g., pivotally) mounted on distal end 26 of elongate member 20 and is movable between an open position, spaced apart from cartridge assembly 32, to a closed position wherein anvil 34 is in close cooperative alignment with cartridge assembly 32, to thereby clamp tissue. Alternatively, the cartridge assembly 32 may pivot with respect to the anvil member. As shown in Fig. 5, the anvil 34 includes an inner fastener forming surface 39 having a plurality of fastener forming pockets 37 (Fig. 5) that are configured to receive fasteners or staples 33 and to form fasteners or staples 33 in a closed configuration when the fasteners or staples are driven against the anvil 34.

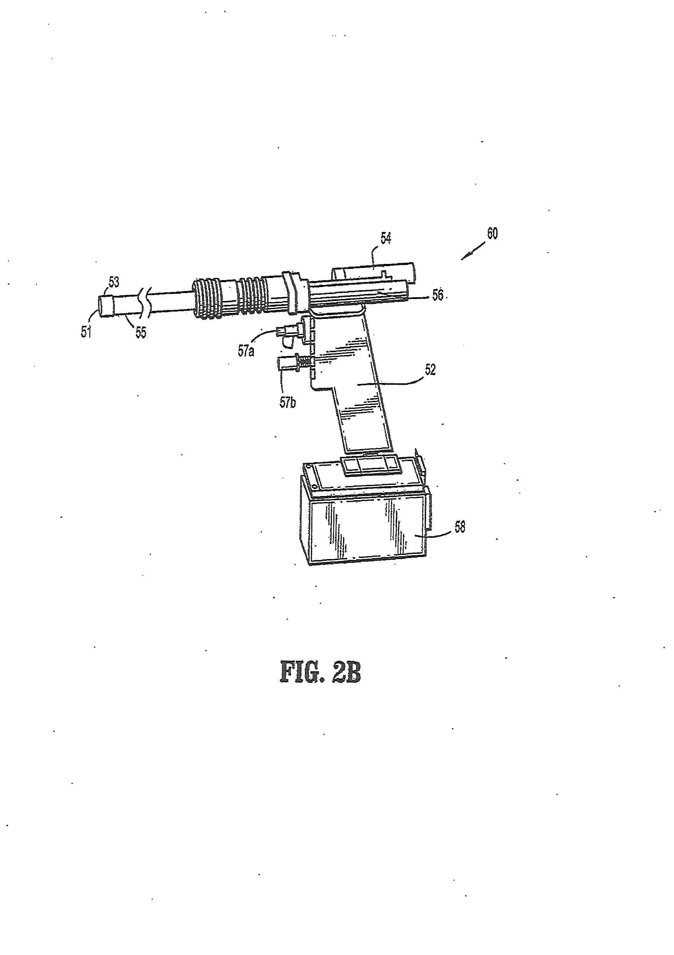

With reference to Figs. 1, 2B and 6, during use, anvil 34 is moved between the open and closed positions by actuator 14. In embodiments, actuator 14 controls rotational movement of an actuation shaft 55 (Fig. 2B) which is disposed within the elongated member 20. The actuation shaft 55 operates to rotate a drive screw 160 (Fig. 6) to initially move the anvil 34 between the open and closed positions. The drive screw 160 also acts to move an actuation sled 140 (Fig. 6) distally through the cartridge assembly 32 to eject the fasteners 33 as described in more detail below with respect to Figs. 6-11 and 25 through 29.

When fasteners or staples 33 are released, they are configured to penetrate tissue that has been clamped between the approximated cartridge assembly 32 and anvil 34. In certain embodiments, cartridge assembly 32 may be a replaceable cartridge so that when all of fasteners 33 have been expelled, cartridge assembly 32 may be replaced with a replacement cartridge (not shown), in order to continue a surgical procedure. Alternatively, the jaw assembly 30 is incorporated into a loading unit that can be replaced after the fasteners are fired. The loading unit may have a body portion that attaches to the elongated member 20.

In other embodiments, jaw assembly 30 may include a pair of jaw members that are configured to utilize laser energy, radio-frequency energy or any other suitable techniques to treat or join tissue. The laser and/or radio frequency energy may be used in combination with cartridge assembly 32 and anvil 34.

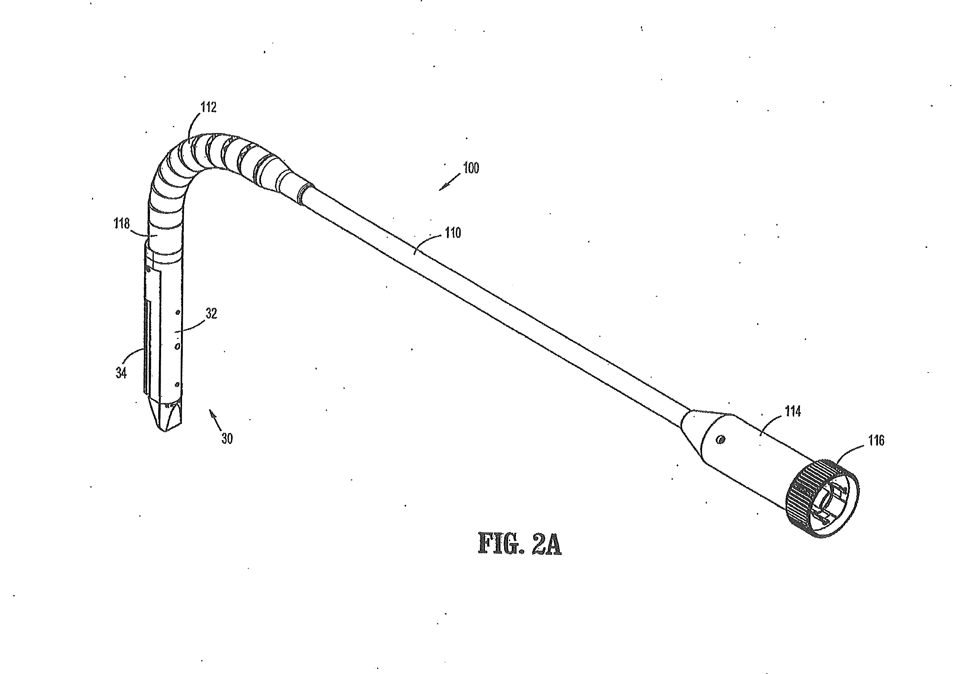

Referring now to Fig. 2A, another embodiment of an elongated member 100 is shown. The elongated member 100 can be attached to a handle assembly 12 or handle assembly 60 or handle assembly 1102. The jaw assembly 30 can be as described in more detail below in Figs. 6 through 11 or Figs. 25 through 29. Elongated member 100 includes a rigid shaft portion 110 on one end and a flexible shaft portion 112 on the other end. Rigid shaft portion 110 includes a straight, rigid configuration that is intended to remain in a fixed longitudinal configuration. A proximal portion 116 of elongated member 100 includes a joining mechanism 114 that is configured to operably couple to joining portion 18 of handle assembly 12, or another handle assembly. It is envisioned that any type of combination of shafts (e.g., flexible and rigid) may be utilized. For example, only a fixed shaft portion 110 may be used and/or only a flexible shaft portion 112 may be used. It is desirable that at least a portion of the elongated member 100 is flexible or otherwise configured for passage through a lumen in a patient's body, such as the esophagus. In certain embodiments, rigid shaft portion 110 may be disposed on the distal portion 118, while flexible shaft portion 112 may be disposed on the proximal portion 116. In embodiments, flexible shaft portion 112 may be steered and/or articulated by a user via controls (not shown) disposed on handle assembly 12, and articulating mechanisms described above. In certain embodiments, the elongated member 100 has a single pivotable member for articulating the jaw assembly. In certain preferred embodiments, the elongated member 100 has a plurality of pivotable members, or is otherwise configured, so that the elongated member 100 is capable of gently curving in one or more directions. Actuators for remotely articulating or changing the shape of the elongated member 100, such as cables and/or linkages, can be provided, or the elongated member may be manipulatable by hand.

Furthermore, an adapter may be configured for being removably attached to the elongated member 100 and to the loading unit or other jaw assembly, or the elongated member 100 itself may form a removable adapter. Such adapters may include an adapter for connection to a manually powered handle assembly. Additionally or alternatively, adapters permitting alternative shaft lengths, rigid or flexible shafts, steerable shafts, shafts in various shapes, etc., can be provided for connecting the loading unit or jaw assembly to a handle assembly. The elongated member 100 can be configured as a removable and replaceable adapter and can include an interface between the proximal end of the shaft and the distal end of the handle, providing coupling means for the particular jaw assembly to be utilized.

Reference may be made to U.S. Patent Application Serial Publication No. 12/622,827, filed on November 20, 2009, entitled "Surgical Console and Hand-held Surgical Device", the entire content of which is hereby incorporated herein by reference, for a detailed discussion of the construction and operation of a motorized handle assembly 60. The motor may be provided in the handle assembly 60 or in a separate unit. The power source for a motorized apparatus may likewise be provided in the handle assembly 60 of the instrument, or in a separate unit.

In embodiments, surgical instrument 10 may include a motorized handle assembly 60 as shown in Fig. 2B. Motorized handle assembly 60 may be disposed at least partially within the housing 16 and includes a control board 52 for controlling motors 54, 56, which impart rotation motion to the actuation shaft 55 and/or other mechanisms for articulating the jaw assembly 30. The motors 54, 56, may be dual-directional motors which impart rotation to the actuation shaft 55. The control board 52 is also coupled to switches 57a and 57b that are actuated by the actuator 14. In addition to being coupled to the switches 57a and 57b, the motors 54 and 56 are also coupled to a power source 58. In embodiments, the power source 58 may be a single direct current removable and rechargeable battery pack. In further embodiments, alternative power sources, including, but not limited to, dual direct current sources or single remote alternating current sources may be used. In the event that the driver device is useable with an alternating current, a transformer may be included between the motors 54, 56 and the power source 58, or an intermediate gearing assembly may be provided between the motors 54, 56 and the actuating shaft 55.

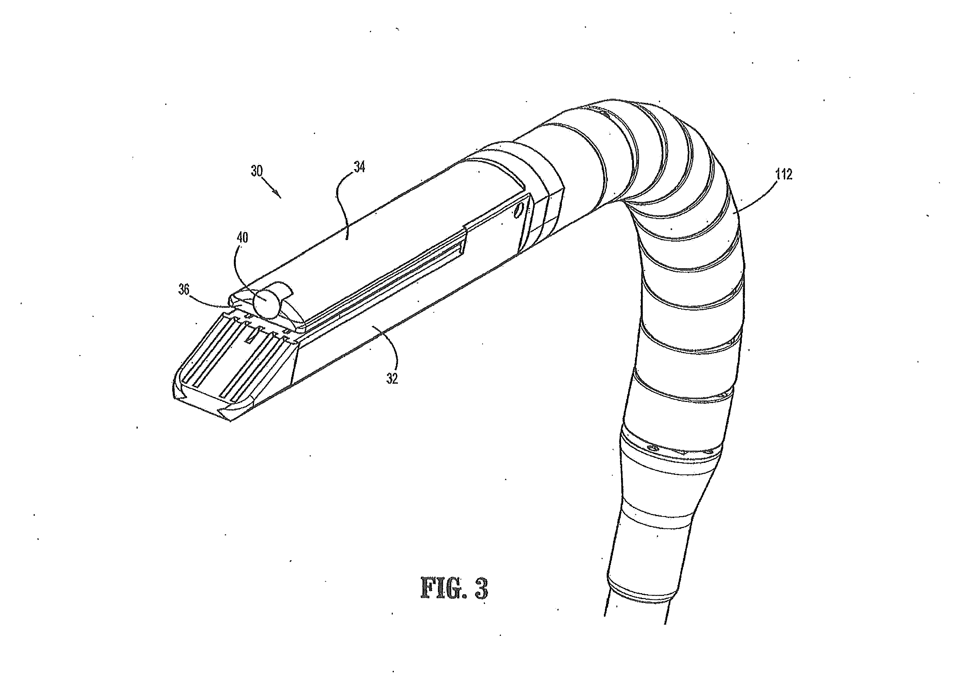

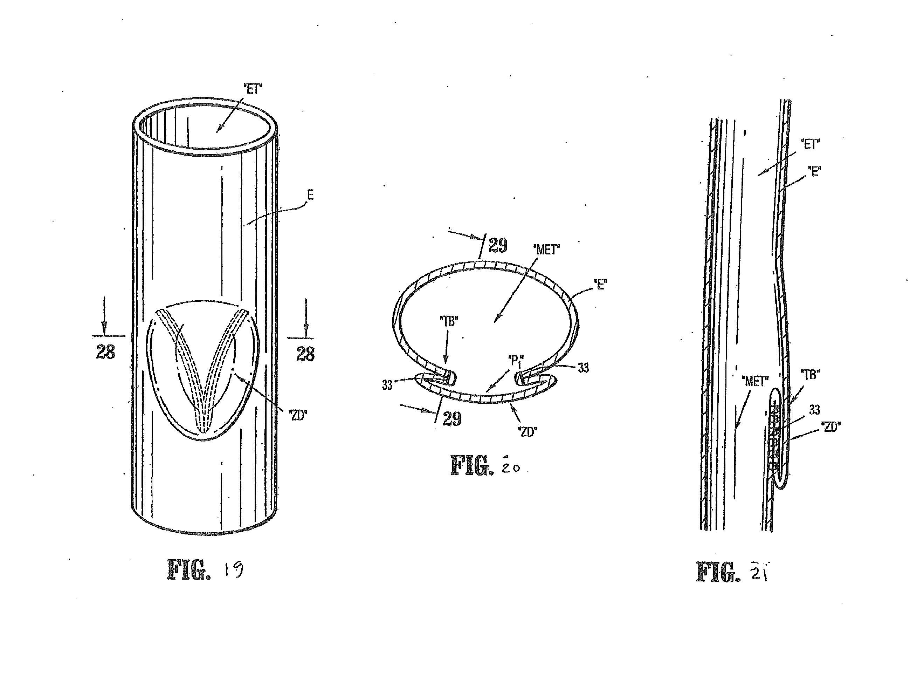

Referring now to Fig. 3, jaw assembly 30 is shown having an image-capturing device, such as an endoscopic camera 40, operably coupled to a distal portion of anvil 34. Endoscopic camera 40 is configured to allow someone to view, from a remote location, an operative area in which the jaw assembly 30 is introduced into. In a procedure for treating a diverticulum, the camera can be used to view a portion of the esophageal track. For example, camera 40 may be configured to view the esophageal tract "ET" (Figs. 19-21) of a patient when jaw assembly 30 is positioned therewithin. Endoscopic camera 40 may be for example, but not limited to, an endoscope or some other kind of scope, a video camera, a photographic camera, an LED-provided camera, or any other suitable image-capturing device. The camera can be attached to an eyepiece or a video monitor or computer monitor. By mounting endoscopic camera 40 directly on jaw assembly 30, endoscopic camera 40 facilitates and ensures a direct and accurate view of the operative site during positioning of instrument 10 during a surgical procedure. Additionally or alternatively, it is envisioned that endoscopic camera 40 may be mounted on cartridge assembly 32 or any other suitable location of surgical instrument 10.

Referring to Figs. 4 and 5, the jaw assembly 30 is shown. Anvil 34 is shown having a distal portion forming a blunt tip 36, fastener pockets 37 defined in the fastener forming surface 39, and a knife slot 38. The blunt tip 36 is at the terminal end of the anvil 34 member and is proximal of the distal end of the cartridge assembly, the distal portion of the staple cartridge extending distally beyond the distal end of the anvil, so that the anvil 34 is shorter than the total length of the cartridge assembly 32. As discussed above, fastener forming pockets 37 are configured to form the fasteners or staples 33 when the anvil 34 is in a closed position. Knife slot 38 is configured to extend up to blunt tip 36 to allow a knife blade 174 (Fig. 6) to cut tissue up to the distal-most fastener forming pocket 37. The cartridge assembly has a distal end defining an angled surface extending from a position proximal of the blunt tip. In this manner, an end of a cut line "CL" (i.e., distal end of the knife slot 38) is close to and proximal to an end of a fastener line "SL" (i.e., distal-most fastener pocket 37). The cut line should be as close as possible to the end of the fastener or staple line. In addition, blunt tip 36 facilitates insertion of the jaw assembly (e.g., anvil 34 and cartridge assembly 32) as deep as possible within a cavity, for example, the Zenker's diverticulum "ZD" (Fig. 20). More specifically, during the procedure, the anvil will be positioned inside the esophageal pocket that forms the diverticulum, whereas the staple cartridge will be positioned outside of that pocket and inside the esophagus. Thus, it is desirable in certain embodiments for the blunt tip to extend beyond the distal end of the cartridge assembly 32. In certain embodiments, the scope or camera may be located on the blunt tip 36, which may extend beyond the distal end of the cartridge assembly 32. It is envisioned that blunt tip 36 may also prevent the distal end of jaw assembly 20 from damaging surrounding tissue when inserted within the esophagus and the Zenker's diverticulum "ZD." Alternatively, the blunt tip 36 may have the shape of a hook, in other embodiments.

Figs. 6-11 illustrate components and operation of a jaw assembly 30. Referring to Fig. 6, an exploded view of the jaw assembly 30 is shown. The jaw assembly 30 is adapted to apply a plurality of linear rows of fasteners or staples 33, which in embodiments may be of various sizes, e.g., about 30 mm in length. The jaw assembly 30 also includes a carrier 31 having an elongate channel 110 having a base 112 and two parallel upstanding walls 114 and 116 which include several mounting structures, such as notches 139, for supporting the cartridge assembly 32 and the anvil 34. A longitudinal passage 111 extends through the elongate channel 110.

With continuing reference to Fig. 6, the distal portion of channel 110 supports cartridge assembly 32 which contains a plurality of surgical fasteners 33 and a plurality of corresponding ejectors or pushers 137. Actuation sled 140 having upstanding cam wedges 144, exerts a fastener driving force on the pushers 137, which drive the fasteners 33 from cartridge assembly 32, as described in more detail below. Cartridge assembly 32 is maintained within channel 110 by lateral struts 136 which frictionally engage the upper surfaces of channel walls 114 and 116, and the frictional engagement of housing tabs, such as tab 138, within notches 139. These structures serve to restrict lateral, longitudinal, and elevational movement of the cartridge assembly 32 within channel 110.

A plurality of spaced apart longitudinal slots 142 extend through cartridge assembly 32 to accommodate the upstanding cam wedges 144 of actuation sled 140. Slots 142 communicate with a plurality of transverse retention slots 146 within which the plurality of fasteners 33 and pushers 137 are respectively supported. The pushers 137 are secured by a pusher retainer 135 disposed below the cartridge assembly 32. The pusher retainer 135 supports and aligns the pushers 134 prior to engagement thereof by the actuation sled 140. During operation, as actuation sled 140 translates through cartridge assembly 32, the angled leading edges of cam wedges 144 sequentially contact pushers 137, causing the pushers to translate vertically within slots 146, urging the fasteners 134 therefrom. The cartridge assembly 32 also includes a longitudinal slot 185 to allow for the knife blade 174 to travel therethrough, as described in more detail below.

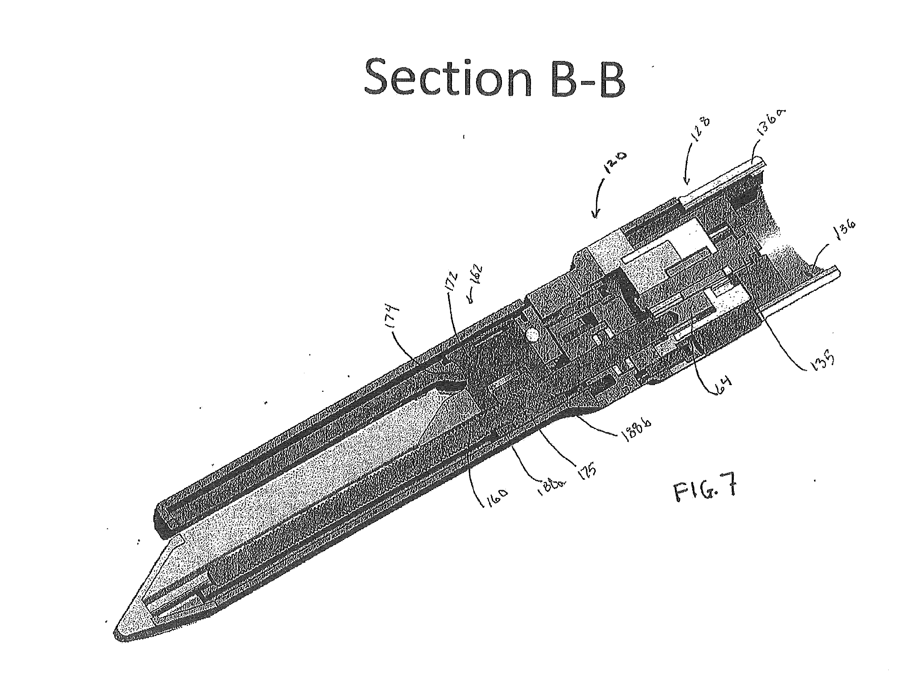

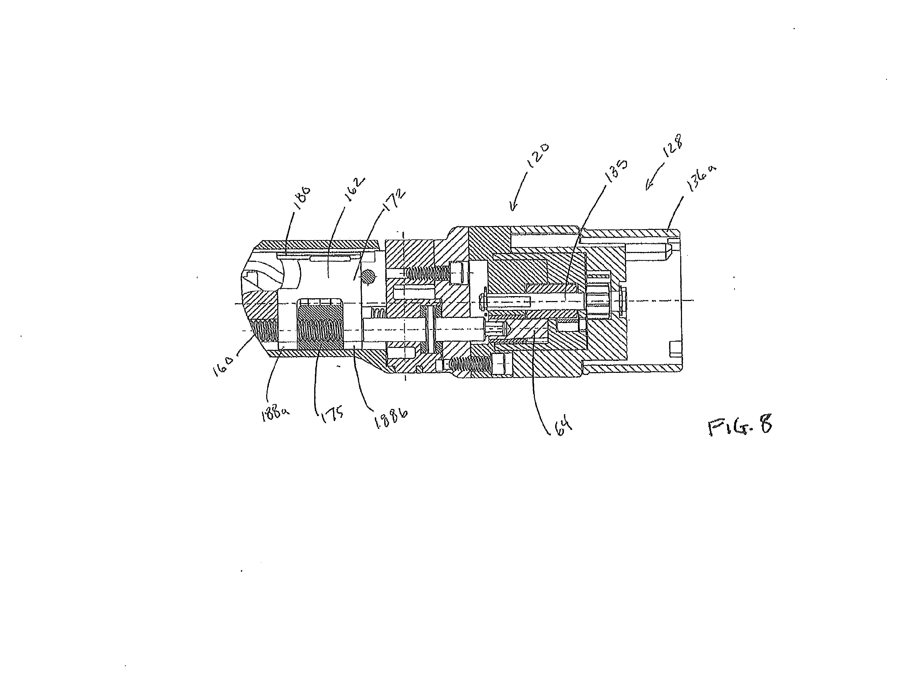

With continuing reference to Fig. 6, the jaw assembly 30 includes an anvil cover 35 disposed over the anvil 34. The anvil cover 35 protects tissue from moving parts along the exterior of anvil 34. The anvil cover 35 includes opposed mounting wings 150 and 152 to align the anvil 34 with the cartridge assembly 32 during closure as shown in Figs. 7 and 8. The anvil 34 along with the cover 35 is configured to remain in an open configuration until closed, as described in more detail below.

The anvil 34 and the carrier 31, including the cartridge assembly 32, are coupled to a mounting portion 120. The mounting portion 120 has a first spacer 121, as shown in Fig. 6. The first spacer 121 has a protrusion 121a and the anvil cover 35 and carrier 31 are attached to the protrusion 121a by screws or the like. A second spacer 122 is attached to the first spacer 121 by screws or the like and a third spacer 123 is attached to the second spacer 122 by screws or the like.

With reference to Figs. 6 and 6a, a coupling member 128 is coupled to the proximal end of the mounting portion 120. The coupling member 128 includes an axial bore 131 defined therethrough. The mounting portion 120 also includes an axial bore 133 defined therein, which is aligned with the axial bore 131 of coupling member 128 when the coupling member 128 is coupled thereto. The bores 131 and 133 are threaded and are dimensioned and configured to be interconnected by a bolt 135. (See Fig. 7)

With continued reference to Figs. 6-9, the coupling member 128 includes three protrusions 136 in sleeve 136a, as shown in Fig. 7, and one or more alignment shafts 129, as shown in Fig. 6. The alignment shafts 129 along with the protrusions 136 are used to align and couple the jaw assembly 30 to the distal end 24 of the elongated member 20. The shafts 129 align the elongated member 20 with the jaw assembly 30 and the protrusions 136 define a conventional bayonet-type coupling which facilitates quick and easy engagement and removal of the jaw assembly 30 from the instrument 10 during a surgical procedure. Once engaged in the distal end 24 of the elongated portion 20, the drive mechanism, e.g., actuation shaft 55, of the instrument 10 is coupled to a drive shaft 64 (see Fig. 7) of the jaw assembly 30, through the elongated member 20 or elongated member 100.

As seen in Fig. 6, jaw assembly 30 further includes an axial drive screw 160 for transmitting the rotational drive forces exerted by the drive shaft 64 to actuation sled 140 during a stapling procedure. As shown in Figs. 6 and 7, the drive shaft 64 is disposed within the mounting portion 120 and includes a proximal portion 65 and a distal portion 67. The proximal portion 65 is configured to be engaged by the actuation shaft 55 extending from the instrument 10, and the distal portion 67 is dimensioned and configured to engage the drive screw 160. As shown in Fig. 2B, the actuation shaft 55 includes a distal engagement portion 53. Engagement portion 53 includes a female connection (e.g., hexagonal) which is dimensioned and configured to engage the proximal portion of the drive shaft 64.

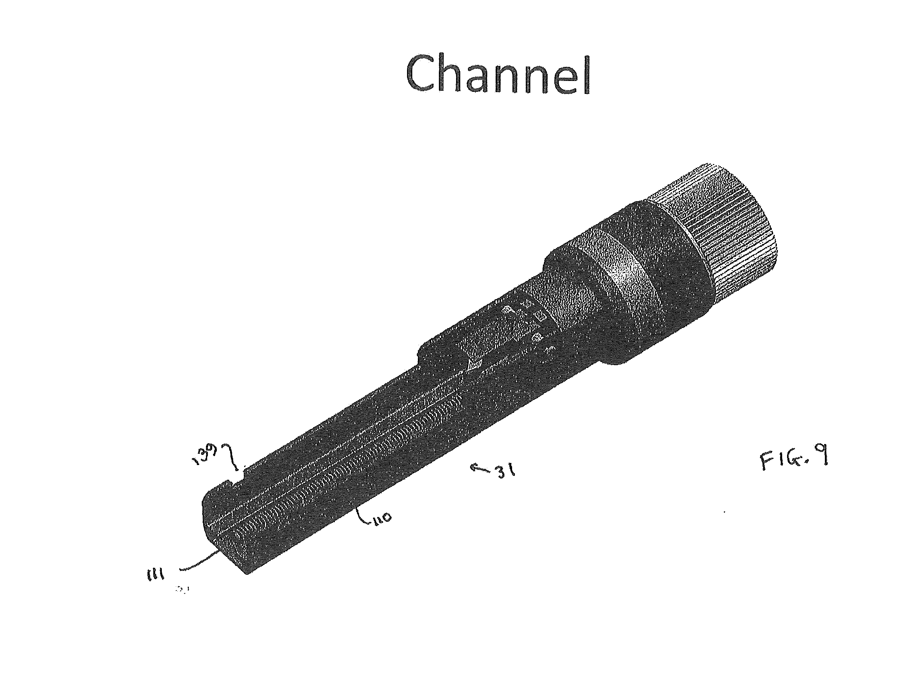

Drive screw 160 includes a threaded portion 160a and a proximal engagement portion 166. Engagement portion 166 includes a male connection 164 which is dimensioned and configured to engage the distal portion of the drive shaft 64. The drive screw 160 is disposed within the longitudinal passage 111 of the carrier 31 as shown in Fig. 9. The drive screw 160 is rotatably secured with respect to the mounting portion 120.

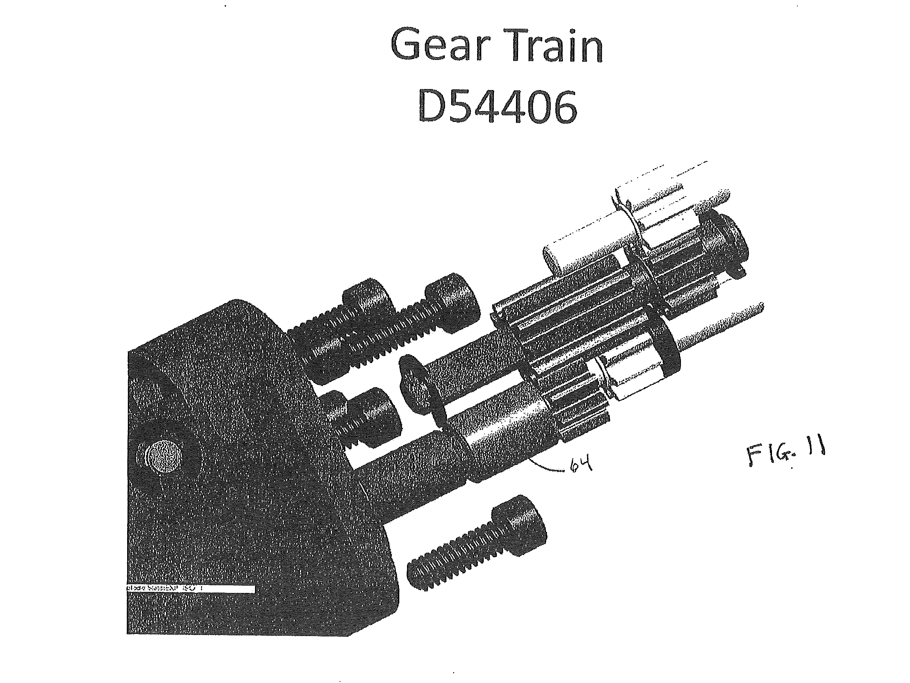

Fig. 11 shows a gear train that can be incorporated in the jaw assembly 30, but preferably at the distal end of the elongated member to adapt the rotational output of the actuation shaft 55 to the input desired for the drive screw 160.

With reference to Figs. 6 and 6a, a drive beam 162 is also disposed within the jaw assembly 30. The drive beam 162 includes a vertical support strut 172 and an abutment surface 176 which engages the central support wedge 145 (Fig. 6a) of actuation sled 140. The drive beam 162 also includes a cam member 180 disposed on top of the vertical support strut 172. (See Fig. 6a). Cam member 180 is dimensioned and configured to engage and translate with respect to an exterior camming surface 182 of anvil 34 to progressively clamp the anvil against body tissue during firing.

A longitudinal slot 184 extends through the anvil 34 to accommodate the translation of the vertical strut 172. After the anvil cover 35 is secured to the anvil 34, a chamber (not shown) defined between an underside of the cover 35 and an upper surface of anvil 34. This allows the cam member 180 to travel in between the cover 35 and anvil 34 during firing.

The drive beam 162 includes a retention foot 188 having a threaded bore 189 defined therethrough. The drive screw 160 is threadably coupled to the drive beam 162 through the bore 189, such that as the drive screw 160 is rotated, the drive beam 162 travels in a longitudinal direction along the axis B-B.

As the drive screw 160 is rotated in a clock-wise direction, the drive beam 162 travels in a distal direction moving the anvil 34 as the cam member 180 pushes down on the camming surface 182 thereof. In this way, the anvil 34 and cartridge assembly are approximated with respect to one another. The drive beam 162 also pushes the sled 140 in the distal direction, which then engage the pushers 134 via the cam wedges 144. The pushers are driven toward the anvil 34 by the wedges 144, ejecting the fasteners 33, and driving the fasteners or staples 33 against the anvil 34.

With reference to Fig. 6a, the drive beam 162 also includes a knife blade 174 for dissecting the fastened tissue. The knife blade 174 travels slightly behind actuation sled 140 during a stapling procedure to form an incision between the rows of fastener body tissue. A follower nut 175 is disposed between two portions 188a 188b of the retention foot 188. In certain embodiments, the drive beam 162 is made from a suitable metal, such as stainless steel, whereas the follower nut 175 is made from plastic.

In certain embodiments, a surgical stapling instrument 200, has a jaw assembly 30 as discussed above (or the jaw assembly discussed below) and includes, instead of the handle assembly 60, a manually powered handle assembly 210. The handle assembly includes a pistol grip body portion 212 having an elongate tubular member 100 extending distally from body portion 212. As described above, cartridge assembly 32 is mounted on a distal end of elongate tubular member 100. Anvil 34 is movably mounted on the distal end of elongate tubular member 100 and is movable between an open position, spaced apart from cartridge assembly 32, to a closed position wherein anvil 34 is in close cooperative alignment with cartridge assembly 32. In other embodiments, the cartridge assembly 32 is movable.

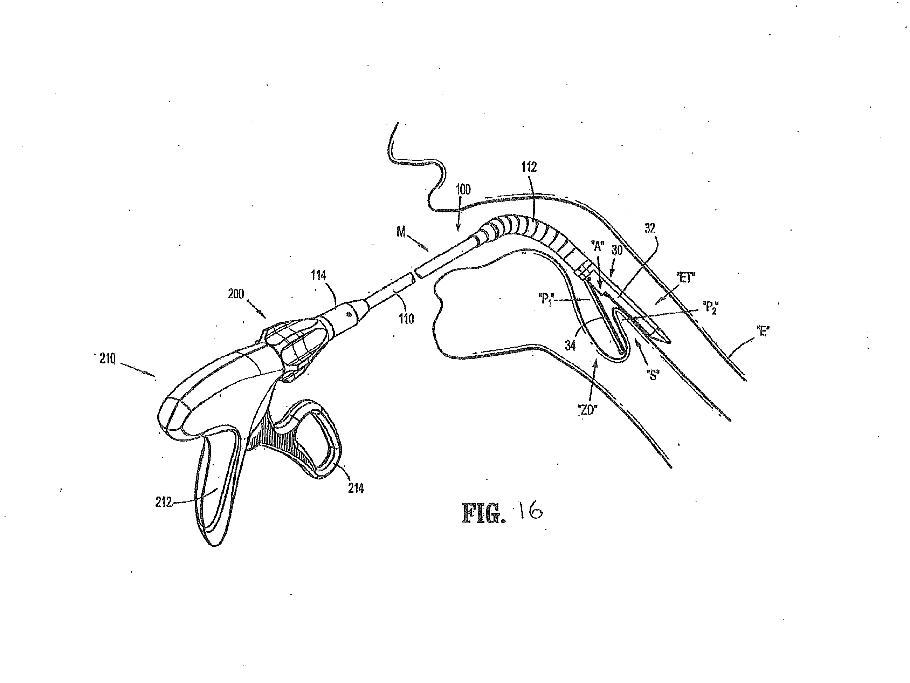

With additional reference to Fig. 16, the manually powered instrument, anvil 34 may be moved between the open and closed positions by an actuator 214 that is pivotally mounted to body portion 212 of surgical stapling instrument 200. Actuator 214 controls rotational movement of the actuation shaft 55, which is disposed within the elongated tubular member 100. The actuation shaft 55, as described above, rotates drive screw 160 (Fig. 6) distally to initially move the anvil 34 between the open and closed positions. The drive screw 160 also moves the actuation sled 140 (Fig. 6) distally through the cartridge assembly 32 to eject the fasteners. Also, as described above, the sled 140 includes the knife blade 174 (Fig. 6) to cut tissue as drive beam 162 translates through the cartridge assembly 32. A rotation knob 200 is provided and configured to orient anvil 34 and cartridge assembly 32 relative to the tissue being fastened.

Briefly, as shown in Figs. 12-15, the Zenker's diverticulum procedure will now be described. An esophagus "E" is shown having an esophageal tract "ET" defined therethrough. A Zenker's diverticulum "ZD" is typically formed on a posterior portion of the esophagus "E" (e.g., towards the back of a neck), which defines a pocket "P1" therein. At a top portion of the Zenker's diverticulum "ZD," a partition "P2" that separates the esophageal tract "ET" and the pocket "P1" is formed between the esophagus "E" and the top portion of the Zenker's diverticulum "ZD." At a bottom portion of the Zenker's diverticulum "ZD," a space "S" is defined between the esophagus "E" and the bottom portion of the Zenker's diverticulum "ZD."

Turning now to Fig. 16, a surgical instrument 200 is shown being positioned within an esophageal tract "E" of a patient. Initially, surgical instrument 200 is inserted into a mouth "M" of a patient "P" in order to access the esophageal tract "E." Surgical instrument 200 is further fed into the esophageal tract "E" until a Zenker's diverticulum is reached. At this point, jaw assembly 30 is opened such that at least one of the jaw members is positioned within the esophageal tract "E" and the other jaw member is positioned within the Zenker's diverticulum "ZD." For example, anvil 34 may be positioned within the Zenker's diverticulum "ZD." and cartridge assembly 32 may be positioned within the esophageal tract "E."

In a first step (Fig. 16), the surgical instrument 200, as described above, is inserted into an esophageal tract "ET" of an esophagus "E" via a mouth "M" of a patient to perform a Zenker's diverticulum treatment. During insertion of surgical instrument 200, a user of surgical instrument 200 may articulate and/or steer flexible shaft 112 by operating handle assembly 210 of surgical instrument 200 towards the Zenker's diverticulum "ZD." In a next step (Fig. 16), jaw assembly 30 is opened such that the jaw members (e.g., cartridge assembly 32 and anvil 34) are positioned about the Zenker's diverticulum "ZD." More specifically, cartridge assembly 32 is positioned within the esophageal tract "ET" and anvil 34 is positioned within the pocket "P1" of the Zenker's diverticulum "ZD." For a more effective surgical procedure, during positioning of the jaw members, a user may urge jaw assembly 30 as far deep as possible, such that partition "P2" is closest to an apex "A" of a pivoting portion of jaw assembly 30. In this configuration, blunt tip 36 of anvil 34 is urged as far as possible within the pocket "P1" of the Zenker's diverticulum "ZD."

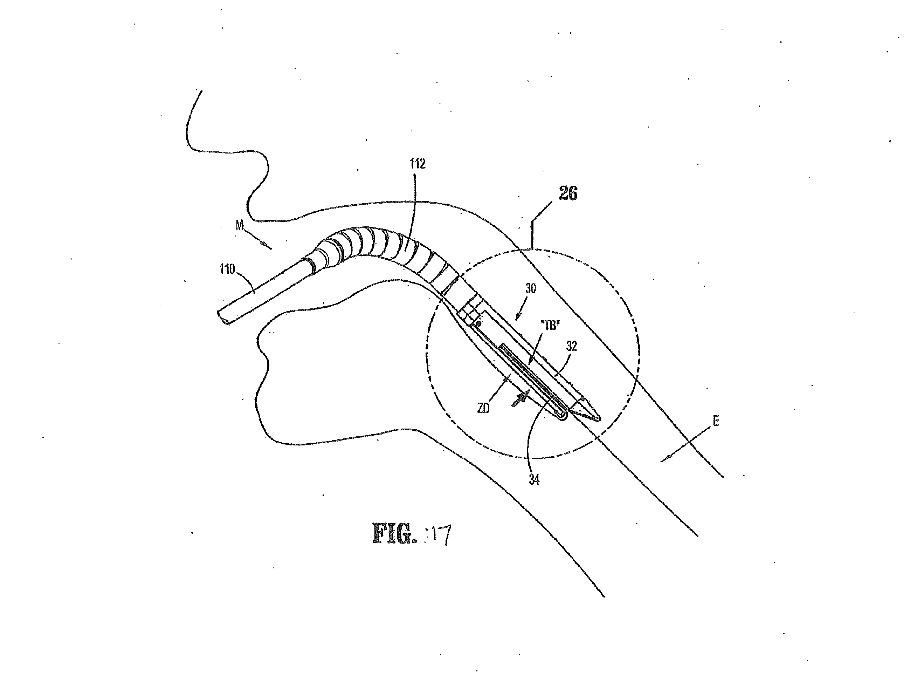

In a next step (Fig. 17), the jaw members (e.g., cartridge assembly 32 and anvil 34) are approximated towards each other such that the pocket "P1" of the Zenker's diverticulum and the esophageal tract "ET" of esophagus "E" are closest to each other. As cartridge assembly 32 and anvil 34 are approximated towards each other, tissue is clamped and the space "S" between the esophagus "E" and the Zenker's diverticulum "ZD" decreases.

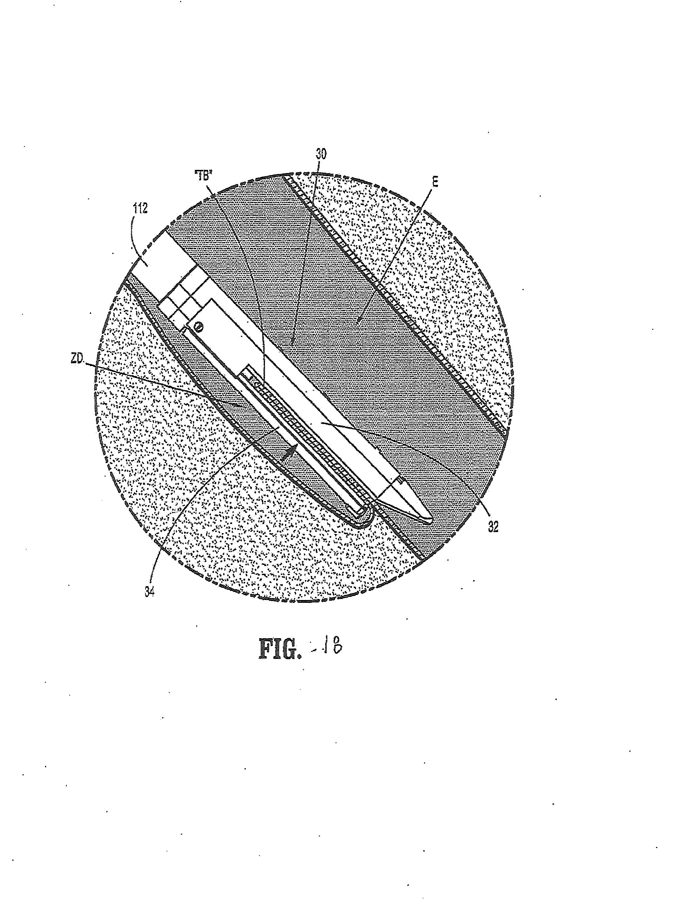

Turning now to Fig. 18, when cartridge assembly 32 and anvil 34 are approximated towards each other and have grasped a tissue bundle "TB," which is comprised of a portion of the esophagus "E" and a portion of the Zenker's diverticulum "ZD" therebetween, a user actuates actuator 214 to provide linear movement of the actuation shaft (not shown) to further move a drive beam or wire (not shown) distally to translate an actuation sled (not shown) such that fasteners 33 are ejected to penetrate through the grasped tissue bundle "TB." In this manner, a wall of the esophagus "E" and a wall of the Zenker's diverticulum "ZD" are joined and attached to each other. The tissue is also severed as described above.

As the knife blade is translated through knife slot 38, the knife blade dissects the tissue bundle "TB" along a mid section to thereby form a modified single esophageal tract "MET," which includes a wall of the esophagus "E" and a wall of the Zenker's diverticulum "ZD." That is, the esophageal tract "ET" is integrated with the pocket "P1" of the esophageal tract "ET." (see Fig. 20). In this configuration, the pocket "P1" of the Zenker's diverticulum "ZD" and the space "S" between the Zenker's diverticulum "ZD" are substantially eliminated.

By eliminating the Zenker's diverticulum "ZD" and providing a modified single esophageal tract "MET," food and other ingested items will continuously travel to the stomach of a patient, instead of being obstructed or trapped within the Zenker's diverticulum "ZD."

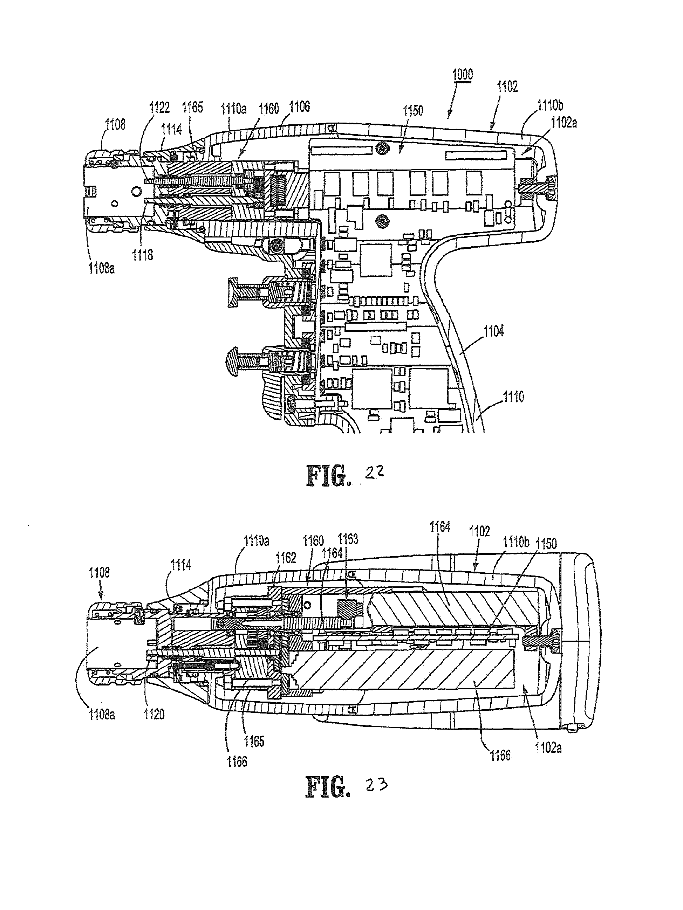

Figs. 22-29 illustrate a powered surgical instrument having a handle assembly 1102 according to another embodiment of the present disclosure. Instrument 1000 is substantially similar to instrument 10 and will only be discussed herein to the extent necessary to identify/describe differences in construction and operation thereof.

Instrument 1000 includes a handle assembly 1102 having a lower housing portion 1104, an intermediate housing portion 1106 extending from and/or supported on lower housing portion 1104, and an upper housing portion 1108 extending from and/or supported on intermediate housing portion 1106. Intermediate housing portion 1106 and upper housing portion 1108 are separated into a distal half-section 1110a that is integrally formed with and extending from the lower portion 1104, and a proximal half-section 1110b connectable to distal half-section 1110a by a plurality of fasteners. When joined, distal and proximal half-sections 1110a, 1110b define a handle assembly 1102 having a cavity 1102a therein in which a circuit board 1150 and a drive mechanism 1160 are situated. The instrument 1000 also includes a power source (not shown), similar to the power source 58 of the instrument 10, which is coupled to the circuit board 1150 and the drive mechanism 1160. Circuit board 1150 is configured to control the various operations of the instrument 1000, in particular, the drive mechanism 1160, as discussed in further detail below.

Lower housing portion 1104 of the instrument 1000 defines an aperture (not shown) formed in an upper surface thereof and which is located beneath or within intermediate housing portion 1106. The aperture of lower housing portion 1104 provides a passage through which wires and other various electrical leads interconnect electrical components (e.g., power source and any corresponding power control circuitry) situated in lower housing portion 1104 with electrical components (e.g., circuit board 150, drive mechanism 160, etc.) situated in intermediate housing portion 1106 and/or upper housing portion 1108.

With reference to Figs. 22 and 23, distal half-section 1110a of upper housing portion 1108 defines a nose or connecting portion 1108a. A nose cone 1114 is supported on nose portion 1108a of upper housing portion 1108. Upper housing portion 1108 of handle assembly 1102 provides a housing in which drive mechanism 1160 is disposed. The drive mechanism 1160 is configured to drive shafts and/or gear components in order to perform the various operations of instrument 1000. In particular, drive mechanism 1160 is configured to drive shafts and/or gear components in order to selectively rotate the jaw assembly 330 about a longitudinal axis "X" (Figs. 33 and 34) relative to handle housing 1102, to move anvil assembly 36 relative to cartridge assembly 32 of the jaw assembly 330, and/or to fire the fasteners 33, and to cut the tissue grasped within the jaw assembly 330.

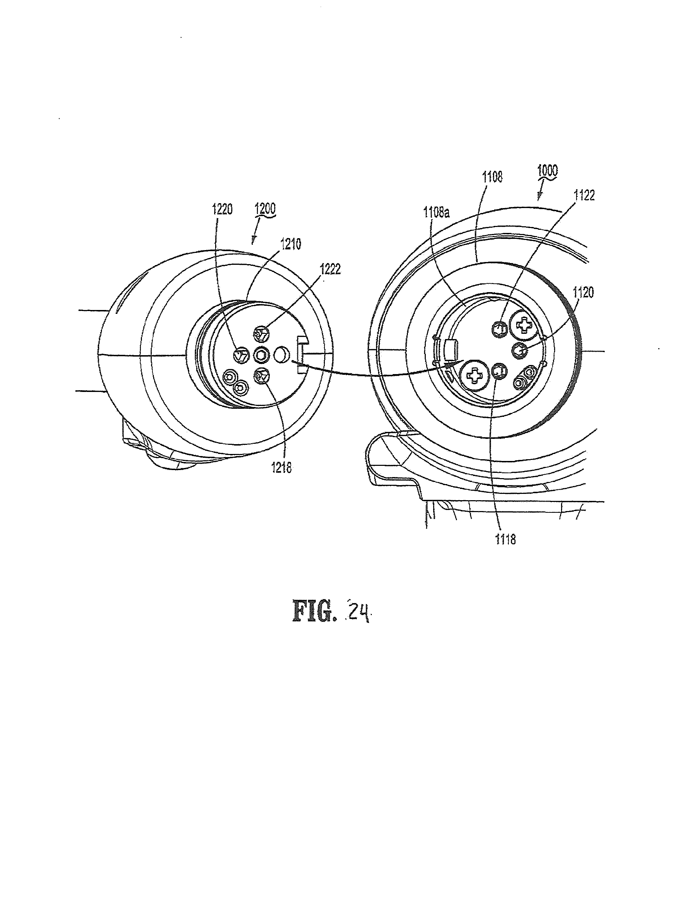

The drive mechanism 1160 includes a selector gearbox assembly 1162 that is located immediately proximal relative to an elongated member 1200 as shown in Fig. 22. Proximal to the selector gearbox assembly 1162 is a function selection module 1163 having a first motor 1164 that functions to selectively move gear elements within the selector gearbox assembly 1162 into engagement with an input drive component 1165 having a second motor 1166. The distal half-section 1110a of upper housing portion 1108 defines a connecting portion 1108a configured to accept a corresponding drive coupling assembly 1210 of the elongated member 1200 as shown in Fig. 24.

The connecting portion 1108a includes a cylindrical recess 1108b that receives a drive coupling assembly 1210 of elongated member 1200. Connecting portion 1108a houses three rotatable drive connectors 1118, 1120, 1122. When elongated member 1200 is mated to instrument 1000, each of rotatable drive connectors: first drive connector 1118, second drive connector 1120, and third drive connector 1122 of instrument 1000 mechanically engages a corresponding rotatable connector sleeve, namely, first connector sleeve 1218, second connector sleeve 1220, and third connector sleeve 1222 of elongated member 1200.

The mating of drive connectors 1118, 1120, 1222 of instrument 1000 with connector sleeves 1218, 1220, 1222 of elongated member 1200 allows rotational forces to be independently transmitted via each of the three respective connector interfaces. The drive connectors 1118, 1120, 1122 of instrument 1000 are configured to be independently rotated by drive mechanism 1160. In this regard, the function selection module 1163 of drive mechanism 1160 selects which drive connector or connectors 1118, 1120, 1122 of instrument 1000 is to be driven by the input drive component 1165 of drive mechanism 1160.

Since each of drive connectors 1118, 1120, 1122 of instrument 1000 has a keyed and/or substantially non-rotatable interface with respective connector sleeves 1218, 1220, 1222 of elongated member 1200, when elongated member 1200 is coupled to instrument 1000, rotational force(s) are selectively transferred from drive mechanism 1160 of instrument 1000 to elongated member 1200.

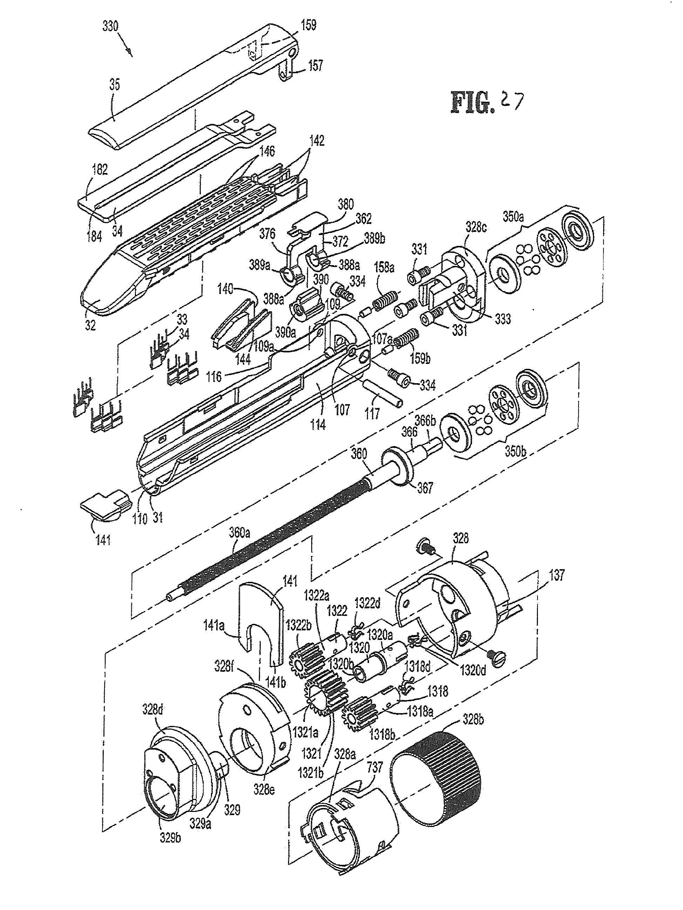

The selective rotation of drive connector(s) 1118, 1120 and/or 1122 of instrument 1000 allows instrument 1000 to selectively actuate different functions of the jaw assembly 330. As will be discussed in greater detail below, selective and independent rotation of second drive connector 120 of instrument 1000 corresponds to the selective and independent opening and closing of jaw assembly 330, and driving of the actuation sled 140 (Fig. 27) component of jaw assembly 330. The selective and independent rotation of first and third drive connectors 1118 and 1122 of instrument 1000 corresponds to the selective and independent rotation of jaw assembly 330 about longitudinal axis "X" relative to handle housing 1102 of instrument 1000.

With reference to Figs. 22 and 23, drive mechanism 1160 includes a selector gearbox assembly 1162; a function selection module 1163, located proximal to the selector gearbox assembly 1162, that functions to selectively move gear elements within the selector gearbox assembly 1162 into engagement with second motor 1166. Thus, drive mechanism 1160 selectively drives one or more of drive connectors 1118, 1120, 1122 of instrument 1000 at a given time.

Figs. 22-29 illustrate the components and operation of a jaw assembly 330 according to another embodiment of the present disclosure. Referring to Fig. 27, an exploded view of the jaw assembly 330 is shown. The jaw assembly 330 is adapted to apply a plurality of linear rows of fasteners or staples 33. The jaw assembly 330 is similar to the jaw assembly 30 with same components identified by same numbers and only the differences between the embodiments are described herein below.

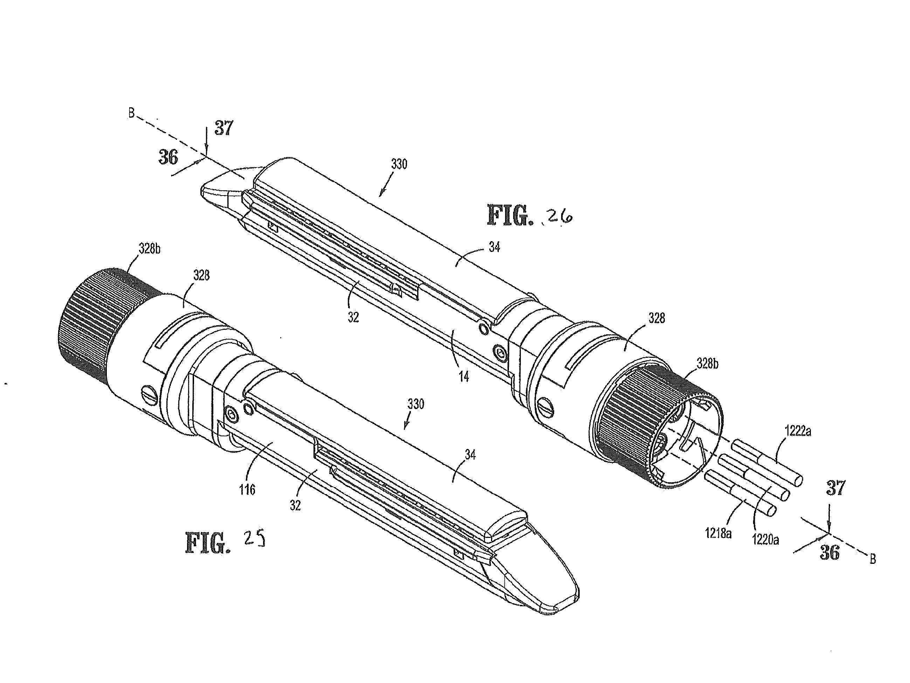

The jaw assembly 330 includes a coupling member 328 for coupling the jaw assembly 330 to the elongated member 1200. The jaw assembly 330 includes a mounting portion 328a and a ribbed sleeve 328b. The coupling member 328 also includes a first, second, and third housing spacers 328c, 328d, 328e. The first housing spacer 328c is coupled to the second housing spacer 328d via a plurality of screws. The first housing spacer 328c includes an engagement portion 333 coupled to carrier 31 via screws 334. The second housing spacer 328d includes a mounting axle 329 having one or more flat surfaces 329a. The coupling member 328 and the mounting portion 328a include one or more J-shaped slots 737 for coupling to a distal end of the elongated member 1200. The elongated member 1200 includes three drive shafts 1218a, 1220a, 1222a, as shown in Fig. 26, which are coupled to the connector sleeves 1218, 1220, 1222 (Fig. 25).

The jaw assembly 330 also includes an axial drive screw 360 for transmitting the rotational drive forces exerted by the drive shaft 1220a to the actuation sled 140 during a stapling procedure. The drive shafts 1218a, 1220a, 1222a, are dimensioned and configured to mechanically engage couplings 1318, 1320, 1322, respectively. Each of the couplings 1318, 1320, 1322 includes a shaft 1318a, 1320a, 1322a, respectively, rotatably disposed within the coupling member 328, in particular spacer housings 328c, 328d and 328e.

A gear 1321 includes an opening 1321a having one or more flat surfaces 1321b on an inner surface thereof. The gear is coupled to the axle 329, with the respective flat surfaces 1321b and 329b mechanically engaging with each thereby preventing rotation motion of the gear 1321 relative to the housing spacers 328d and 328e. The housing spacer 328c also includes an opening 329b defined therethrough. The opening 329b also passes through the axle 329 and allows for the coupling 1320 to pass therethrough and to couple to the drive screw 360. Each of the couplings 1318 and 1322 also includes a gear 1318b and 1322b at a distal end thereof. Each of the couplings 1318, 1320, 1322 also include an opening 1318c 1320c, at its proximal end and a spring 1318d, 1320d, 1322d disposed therein. The springs 1318d, 1320d, 1322d frictionally engage the drive shafts 1218a, 1220a, 1222a.

Drive screw 360 includes a threaded portion 360a and a proximal engagement portion 366. Engagement portion 366 includes a multi-faceted or non-circular male connection 366a (e.g., hexagonal) which is dimensioned and configured to engage male connection 1320b at a distal end of the coupling 1320. The drive screw 360 is disposed within the longitudinal slot 111 of the carrier 31 as shown in Fig. 35. The drive screw 360 is rotatably secured at its engagement portion 366 via a thrust plate 141. The thrust plate 141 is inserted through a slit 328f in the second housing spacer 328e, as shown in Fig. 35. In particular, the thrust plate 141 includes a pair of teeth 141a and 141b that engage the drive screw 360 between the engagement portion 366 and a protrusion 367, thereby preventing lateral, longitudinal, and elevational movement and allowing only for rotation of the drive screw 360 about a longitudinal axis B-B (Fig. 26).

Rotation of the drive screw 360 actuates a drive beam 362 in a similar manner as described above. The drive beam 362 includes a vertical support strut 372 and an abutment surface 376 which engages the actuation sled 140. The drive beam 362 also includes a cam member 380 disposed on top of the vertical support strut 372. Cam member 380 is dimensioned and configured to engage and translate with respect to an exterior camming surface 182 of anvil 34 to progressively clamp the anvil against body tissue during firing.

The drive beam 362 includes a distal retention foot 388a and a proximal retention foot 388b, each having a bore 389a and 389b defined therethrough. The bores 389a and 389b may be either threaded or smooth to provide for travel along the drive screw 360 which passes therethrough. A travel nut 390 having a threaded bore 390a therethrough is disposed between the distal and proximal retention feet 388a and 388b. The drive screw 360 is threadably coupled to the travel nut 390 through the bore 390a, such that as the drive screw 360 is rotated, the travel nut 390 travels in a longitudinal direction along the axis B-B and also engaging the feet 388a and 388b. As the drive screw 360 is rotated in a clock-wise direction, the travel nut 390 and the drive beam 362 travel in a distal direction closing the anvil 34 as the cam member 380 pushes down on the camming surface 182 thereof. The drive beam 362 also pushes the sled 140 in the distal direction, which then engages the pushers 34 via the cam wedges 144 to eject the fasteners 33.

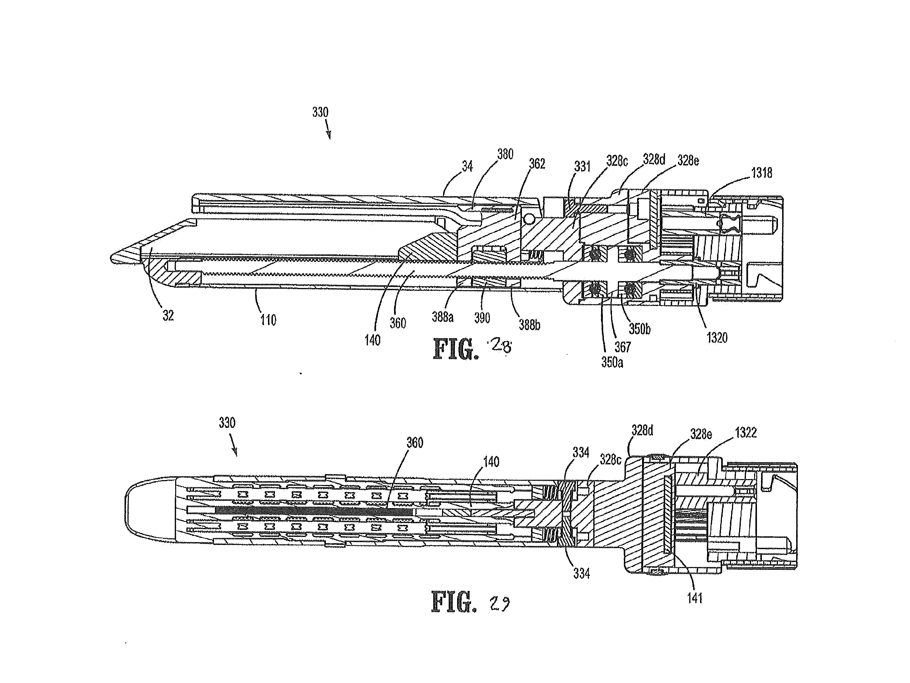

The drive screw 360 is rotationally disposed within the coupling member 328 via first and second bearings 350a and 350b. The bearings 350a and 350b are frictionally fitted to the driver screw 360 at distal and proximal sides of the protrusion 367. The bearings 350a and 350b are also frictionally fitted within the second housing spacer 328b as shown in Figs. 27 and 28. This allows the drive screw 360 to be rotated relative to the coupling member 328. Since the components of the jaw member 330, including the anvil 34 and the cartridge assembly 32, are coupled to the coupling member 328, the drive screw 360 and the jaw assembly 330 are free to rotate relative to each other.

As described above, rotation of the drive screw 360 relative to the coupling member 328 is accomplished via the drive shaft 1220a. Rotation of the coupling member 328 allows for rotation of the jaw assembly 330 about the longitudinal axis B-B. The drive shafts 1218a and 1222a engage the couplings 1318 and 1322, respectively. The rotation of the drive shafts 1218a and 1222a then rotates the gears 1318b and 1322b, respectively, which are mechanically engaged with the gear 1321 coupled to the second housing spacer 328e. The gear 321 acts as a sun gear, with the gears 1318b and 1322b acting as planetary gears allowing for the jaw assembly 330 to be rotated about the longitudinal axis B-B. This motion provides for adjustment of the position of the jaw assembly 330 without rotating the entire adapter assembly 1200.

In further embodiments, the instruments of Figs. 1 through 29 can have removable and replaceable jaw assemblies and/or elongated members. The handle assembly may be manually powered or motorized.

Each of the embodiments described above are provided for illustrative purposes only and it is within the concept of the present disclosure to include modifications and varying configurations without departing from the scope of the disclosure that is limited only by the claims included herewith.

## Claims

1. A surgical instrument for performing an endolumenal surgical procedure, comprising:
a handle assembly,
an elongated member operably coupled to the distal end of the handle assembly and
a jaw assembly having a pair of jaw members, one or both of the jaw members defining a knife slot adapted to receive a knife blade, one of the pair of jaw members including an anvil and the other of the pair of jaw members including a cartridge assembly, the pair of jaw members being movable relative to one another so that the anvil and cartridge assembly can be moved to an approximated position, the anvil and cartridge assembly each having distal portions, the distal portion of the cartridge assembly extending distally beyond the distal portion of the anvil.

2. The surgical instrument according to claim 1, wherein the elongated member and jaw assembly are configured to be inserted into the esophagus of a patient.

3. The surgical instrument according to claim 2, wherein the jaw assembly is configured to engage a portion of the esophagus to treat a diverticulum in the esophagus.

4. The surgical instrument according to any preceding claim, wherein the elongated member is flexible.

5. The surgical instrument according to any preceding claim, wherein the elongated member has an articulating portion.

6. The surgical instrument according to any preceding claim, further comprising an endoscopic camera that is operably coupled to a distal portion of at least one of the jaw members.

7. The surgical instrument according to claim 6, wherein the endoscopic camera is positioned on the at least one of the jaw members to provide an endoscopic view of the esophageal tract.

8. The surgical instrument according to claim 6, wherein the camera is positioned adjacent the anvil.

9. The surgical instrument according to any preceding claim, wherein the distal portion of the anvil forms a blunt tip.

10. The surgical instrument according to claim 9, wherein the knife slot terminates at position substantially aligned with a distal-most fastener forming pockets of the anvil.
